(19) Europäisches Patentamt
European Patent Office
Office européen des brevets

(11) Numéro de publication : **0 425 324 B1**

# (12) FASCICULE DE BREVET EUROPEEN

(45) Date de publication du fascicule du brevet :
**27.10.93 Bulletin 93/43**

(51) Int. Cl.$^5$ : **A61K 7/42**

(21) Numéro de dépôt : **90402360.3**

(22) Date de dépôt : **24.08.90**

## (54) Composition pour la coloration de la peau à base de dérivés indoliques et d'hydroxyacétone.

(30) Priorité : **29.08.89 FR 8911351**

(43) Date de publication de la demande :
**02.05.91 Bulletin 91/18**

(45) Mention de la délivrance du brevet :
**27.10.93 Bulletin 93/43**

(84) Etats contractants désignés :
**AT BE CH DE DK ES FR GB GR IT LI NL SE**

(56) Documents cités :
**EP-A- 0 239 826**
**FR-A- 1 293 290**

(73) Titulaire : **L'OREAL**
**14, Rue Royale**
**F-75008 Paris (FR)**

(72) Inventeur : **Cotteret, Jean**
**15 Allée des Meuniers**
**F-78480 Verneuil-sur-Seine (FR)**
Inventeur : **Hourseau, Colette**
**15, rue Gambetta**
**F-95330 La Frette-sur-Seine (FR)**

(74) Mandataire : **Casalonga, Axel et al**
**BUREAU D.A. CASALONGA - JOSSE**
**Morassistrasse 8**
**D-80469 München (DE)**

EP 0 425 324 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention est relative à l'association de dihydroxyacétone et de dérivés indoliques pour conférer à la peau une coloration similaire à la coloration conférée à la peau par un bronzage naturel, ainsi qu'aux compositions et aux procédés mis en oeuvre.

La dihydroxyacétone ou DHA est connue depuis de nombreuses années dans son application à la coloration de la peau. Ainsi, le brevet FR 1 293 290 a pour objet la dihydroxyacétone et son utilisation dans des agents de bronzage artificiel.

Il a également été proposé de colorer la peau en utilisant des composés indoliques et en particulier le 5,6-dihydroxyindole et certains de ses dérivés, ce qui fait l'objet de l'EP-A-239 826.

Ces différentes possibilités de conférer à la peau une coloration présentent cependant chacune des inconvénients.

La montée de la coloration par la DHA, due essentiellement à une réaction de type MAILLARD entre la DHA et les acides aminés de la peau, est lente. Par ailleurs, la coloration obtenue est généralement assez jaune et relativement éloignée de celle d'un bronzage naturel.

D'autre part, la coloration obtenue avec les dérivés indoliques précités, est également éloignée des nuances d'un bronzage naturel et présente, par ailleurs, l'inconvénient de mal résister à l'eau.

Les inventeurs ont découvert, de façon surprenante, qu'en associant la dihydroxyacétone à certains dérivés indoliques, il était possible d'obtenir une coloration intense se développant rapidement dans le temps, beaucoup plus proche, en nuance, de la coloration conférée à la peau par un bronzage naturel, que les colorations obtenues avec chacun des composés pris isolément. La coloration ainsi obtenue présente, par ailleurs, une très bonne ténacité dans le temps et en particulier au lavage.

Un objet de l'invention est donc constitué par l'association de dihydroxyacétone et d'au moins un dérivé indolique, pour conférer à la peau une coloration similaire à celle résultant d'un bronzage naturel.

Un autre objet de l'invention est constitué par des compositions cosmétiques destinées à une application topique, contenant une telle association.

L'invention a également pour objet un procédé permettant de conférer à la peau une coloration similaire à celle résultant d'un bronzage naturel de la peau, par application de l'association précitée.

L'invention concerne enfin des dispositifs à plusieurs compartiments, contenant les différents composants de l'association définie ci-dessus.

D'autres objets de l'invention apparaîtront à la lecture de la description et des exemples qui suivent.

L'association, conforme à l'invention, destinée à conférer à la peau une coloration similaire à la coloration résultant du bronzage naturel, est essentiellement caractérisée par le fait qu'elle comprend :

un composant (A) contenant dans un milieu approprié pour une application topique, de la dihydroxyacétone, et

un composant (B) contenant dans un milieu approprié pour la peau, un colorant indolique, répondant à la formule :

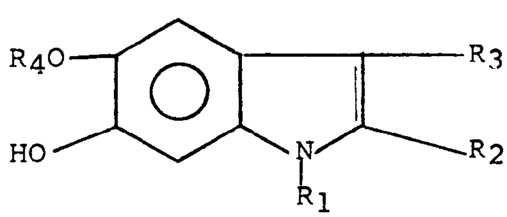

(I)

dans laquelle $R_1$, $R_2$, $R_3$ et $R_4$ désignent, indépendamment l'un de l'autre, hydrogène ou un groupement alkyle en $C_1$-$C_4$, à la condition que lorsque $R_4$ représente un groupement alkyle en $C_1$-$C_4$, $R_1$, $R_2$ et $R_3$ désignent hydrogène;

ainsi que les sels d'addition de ces composés;

les composants (A) et (B) faisant partie d'une même composition ou étant destinés, soit à être mélangés tout juste avant l'emploi, soit à être appliqués de façon successive ou décalée dans le temps.

Les composés particulièrement préférés, répondant à la formule (I), sont choisis parmi le 5,6-dihydroxyindole, le 2-méthyl 5,6-dihydroxyindole, le 3-méthyl 5,6-dihydroxyindole et le 2,3-diméthyl 5,6-dihydroxyindole.

Une première variante de l'invention consiste à utiliser l'association conforme à l'invention, sous forme d'une composition unique, cette composition unique étant, soit préparée et stockée, soit mélangée tout juste avant l'emploi.

Dans ce cas, la dihydroxyacétone est utilisée dans des proportions comprises entre 0,1 et 10% en poids

et en particulier entre 1 et 6%, par rapport au poids total de la composition, contenant les composants (A) et (B), et les dérivés indoliques sont présents dans des proportions comprises entre 0,1 et 10%, et de préférence entre 0,1 et 5%, par rapport au poids total de la composition contenant les composants (A) et (B).

Selon une seconde variante, les composants (A) et (B) sont appliqués, soit de façon successive, soit de façon décalée dans le temps. Dans ce cas, la dihydroxy acétone est présente dans le composant (A) dans des proportions comprises de préférence entre 0,1 et 10% en poids par rapport au poids total du composant (A) et en particulier entre 1 et 6%.

Le ou les dérivés indoliques sont présents dans le composant (B) dans des proportions de préférence comprises entre 0,1 et 10% en poids et en particulier entre 0,1 et 5% en poids par rapport au poids total du composant (B).

Le rapport en poids entre le dérivé indolique de formule (I) et la dihydroxyacétone, est généralement supérieur à 0,01.

Le milieu cosmétiquement acceptable approprié pour une application topique, comprend généralement de l'eau, un mélange d'eau et d'un ou plusieurs solvant(s) organique(s), un mélange de solvants organiques ou encore des corps gras, cosmétiquement acceptables.

Ces compositions peuvent éventuellement être pressurisées dans les dispositifs aérosols en présence d'un agent propulseur, éventuellement en présence d'un générateur de mousse.

Les solvants utilisables sont choisis parmi les alcools inférieurs en $C_1$-$C_4$, tels que l'alcool éthylique, l'alcool isopropylique; les monoalcools saturés à longue chaîne ayant 10 à 18 atomes de carbone; les polyols ayant 2 à 8 atomes de carbone parmi lesquels on peut citer le glycérol; les alkylèneglycols comme l'éthylèneglycol, le propylèneglycol, le diéthylène glycol; les éthers de glycols parmi lesquels on peut citer les monoéthylèneglycol monoalkyléthers, les diéthylèneglycol monoalkyléthers, les triéthylène glycol monoalkyléthers, dans lesquels le groupement alkyle a de préférence 1 à 4 atomes de carbone, tels que par exemple l'éthylèneglycol monoéthyléther, l'éthylèneglycol monobutyléther, le diéthylèneglycol monoéthyléther; les esters acétiques de monoalkyl($C_1$-$C_3$)éthers de l'éthylèneglycol, tels que l'acétate de monométhyléther de l'éthylèneglycol et l'acétate de monoéthyléther de l'éthylèneglycol; les esters d'acides gras saturés ayant de préférence 14 à 16 atomes de carbone et d'alcools saturés ayant de 1 à 4 atomes de carbone, parmi lesquels on peut citer le myristate d'isopropyle et le palmitate d'isopropyle.

Parmi les solvants énumérés ci-dessus, on préfère l'alcool éthylique, l'alcool cétylique, le propylèneglycol, l'éthylèneglycol monoéthyléther et l'éthylèneglycol monobutyléther.

Les solvants, lorsqu'ils sont utilisés dans le milieu aqueux, sont présents de préférence dans des proportions comprises entre 0,5 et 75% en poids et en particulier entre 2 et 50% en poids par rapport au poids total de la composition globale ou de chacun des composants (A) et (B).

Comme corps gras utilisables, on peut citer :

- les huiles et cires d'origine minérale, animale ou végétale,
- les huiles de silicone,
- les acides gras,
- les alcools gras, tels que laurylique, cétylique, myristylique, stéarylique, palmitylique, oléylique,
- les esters d'acides gras, tels que le monostéarate de glycérol, le monostéarate de polyéthylèneglycol,
- les triglycérides d'acides gras en $C_6$-$C_{18}$.

Le milieu approprié pour une application topique peut être épaissi ou non. Pour l'épaissir, on peut utiliser les agents épaississants ou gélifiants bien connus dans l'état de la technique, tels que plus particulièrement la gomme de guar, les hétérobiopolysaccharides, tels que la gomme de xanthane et les scléroglucanes, les dérivés de cellulose, tels que la méthylcellulose, l'hydroxyéthylcellulose, l'hydroxypropylméthylcellulose, les sels de sodium de la carboxyméthylcellulose et les polymères d'acides acryliques préférentiellement réticulés.

Lorsque le milieu renferme de l'eau, le pH de la composition unique renfermant les composants (A) et (B) peut varier entre 3 et 10 et il est de préférence compris entre 3 et 7.

Lorsque les composants (A) et (B) sont utilisés séparément, le pH du composant (A) peut varier entre 3 et 10 et il est plus particulièrement compris entre 3 et 7, tandis que le pH du composant (B) peut varier entre 3 et 10 et il est compris plus particulièrement entre 4 et 7.

Les compositions constituées, soit par l'un et/ou l'autre des composants (A) et (B) ou par la composition contenant les deux composants (A) et (B), peuvent être utilisées sous forme de crèmes, de liquides plus ou moins épaissis, de dispersions vésiculaires de lipides amphiphiles ioniques ou non ioniques, d'huiles ou d'aérosols.

Les dispersions vésiculaires de lipides amphiphiles ioniques ou non-ioniques peuvent être préparées selon des procédés connus.

On peut, par exemple, faire gonfler les lipides dans une solution aqueuse pour former des sphérules dispersées dans le milieu aqueux comme décrit dans l'article BANGHAN, STANDISH et WATKINS, J. mol. Biol.,

13, 238 (1965) ou dans les brevets FR-2 315 991 et 2 416 008 de la demanderesse.

Ces compositions peuvent également contenir tous autres adjuvants utilisés habituellement dans les compositions cosmétiques pour la peau et plus particulièrement des agents tensio-actifs, de préférence non ioniques, des agents hydratants, adoucissants, des vitamines, des opacifiants, des parfums ou des agents conservateurs.

Les composants (A) et/ou (B) constituant la composition peuvent également contenir des filtres solaires filtrant le rayonnement UVB et/ou UVA.

A titre de filtres solaires, on peut citer les dérivés de l'acide cinnamique, le benzylidènecamphre et ses dérivés, l'acide paraaminobenzoïque et ses dérivés, les dérivés de l'acide salicylique, les dérivés de benzophénone, les homosalates, les dérivés du dibenzoylméthane.

Ces filtres sont présents dans des proportions allant de 0,2 à 10% en poids par rapport au poids du constituant (A) et/ou (B).

Les solvants, corps gras ou adjuvants utilisés dans les compositions de l'invention, ne doivent comporter ni groupements amine primaire, ni groupements oxydants.

Lorsque les composants (A) et (B) sont stockés séparément, l'association conforme à l'invention peut être conditionnée dans un dispositif à plusieurs compartiments encore appelé "kit" ou "nécessaire de coloration de la peau", comportant un premier compartiment contenant le composant (A) renfermant la dihydroxyacétone dans un milieu approprié pour une application topique et un second compartiment contenant le composant (B) renfermant, dans 'un milieu approprié pour une application topique, au moins un dérivé indolique de formule (I).

Une variante de ce dispositif peut consister à l'équiper d'un ensemble distributeur à double compartiment, tel que décrit par exemple par la demanderesse dans la demande de brevet français n° 2 586 913 et comportant deux poches séparées réunies dans un étui souple, les deux poches renfermant, pour l'une d'entre elles, le composant (A) tel que défini ci-dessus , et l'autre poche renfermant le composant (A) tel que défini ci-dessus.

Le procédé de traitement, en vue de conférer à la peau une coloration similaire à la coloration résultant d'un bronzage naturel de celle-ci, est essentiellement caractérisé par le fait que l'on applique le composant (A) et le composant (B) sur la peau, soit au moyen d'une seule composition éventuellement préparée tout juste avant l'emploi, soit en appliquant de façon successive le composant (A) et le composant (B) sur les parties de la peau destinées à être colorées, cette application pouvant éventuellement être décalée dans le temps de quelques minutes à plusieurs heures.

Conformément au procédé de l'invention, le composant (A) peut être appliqué avant ou après l'application du composant (B).

Les exemples suivants sont destinés à illustrer l'invention sans pour autant présenter un caractère limitatif.

EXEMPLE 1

On prépare la composition suivante :

| | | | |
|---|---|---|---|
| - 5,6-dihydroxyindole | | | 2,0 g |
| - Dihydroxyacétone | | | 5,0 g |
| - Alcool éthylique | | | 15,0 g |
| - Dérivé de gomme de guar vendu sous la dénomination JAGUAR HP60 par la Société CELANESE | | | 1,0 g |
| - Conservateur | qs | | |
| - Triéthanolamine | qs | pH = 8,5 | |
| - Eau déminéralisée | | qsp | 100,0 g |

Cette composition est appliquée sur une peau non bronzée. La coloration monte rapidement (dès la première heure qui suit l'application, on note une coloration de la peau) et après 5 heures, on obtient une coloration intense très proche en nuance d'un bronzage naturel et qui résiste au lavage.

EXEMPLE 2

On prépare la composition suivante :

| | | | |
|---|---|---|---|
| - 3-méthyl 5,6-dihydroxyindole | | | 0,5 g |
| - Dihydroxyacétone | | | 5,0 g |
| - Alcool éthylique | | | 15,0 g |
| - Dérivé de gomme de guar vendu sous la dénomination JAGUAR HP60 par la Société CELANESE | | | 1,0 g |
| - Conservateur | qs | | |
| - Triéthanolamine | qs | pH = 8,5 | |
| - Eau déminéralisée | | qsp | 100,0 g |

Cette composition permet d'obtenir sur une peau non bronzée une coloration très intense correspondant à un bronzage présentant une légère composante rouge.

EXEMPLE 3

On prépare les compositions suivantes :

COMPOSITION (A) :

| | | | |
|---|---|---|---|
| - Dihydroxyacétone | | | 10,0 g |
| - Alcool éthylique | | | 15,0 g |
| - Gomme de xanthane | | | 1,0 g |
| - Conservateur | qs | | |
| - Triéthanolamine | qs | pH = 8,5 | |
| - Eau déminéralisée | | qsp | 100,0 g |

COMPOSITION (B) :

| | | | |
|---|---|---|---|
| - 5,6-dihydroxyindole | | | 4,0 g |
| - Alcool éthylique | | | 15,0 g |
| - Gomme de xanthane | | | 1,0 g |
| - Conservateur | qs | | |
| - Triéthanolamine | qs | pH = 8,5 | |
| - Eau déminéralisée | | qsp | 100,0 g |

On mélange des quantités égales en poids des compositions (A) et (B), puis on applique ce mélange sur la peau. On observe une coloration identique à celle obtenue par utilisation de la composition de l'exemple 1.

EXEMPLE 4

On prépare la composition suivante :

| | | | |
|---|---|---|---|
| - 3-méthyl 5,6-dihydroxyindole | | | 0,6 g |
| - Alcool éthylique | | | 15,0 g |
| - Gomme de xanthane | | | 1,0 g |
| - Conservateur | qs | | |
| - Triéthanolamine | qs | pH = 8,5 | |
| - Eau déminéralisée | | qsp | 100,0 g |

On mélange des quantités égales en poids de cette composition avec la composition (A) de l'exemple précédent, puis on applique ce mélange sur la peau. On observe une coloration identique à celle obtenue par utilisation de la composition de l'exemple 2.

EXEMPLE 5

On prépare la composition suivante :

| | | | |
|---|---|---|---|
| - Dihydroxyacétone | | | 5,0 g |
| - 5,6-dihydroxyindole | | | 2,0 g |
| - Alcool éthylique | | | 15,0 g |
| - Gomme de xanthane | | | 1,0 g |
| - Conservateur | qs | | |
| - Eau déminéralisée | | qsp | 100,0 g |

Le pH spontané de cette composition est voisin de 5,5.

Cette composition permet d'obtenir sur peau non bronzée une coloration identique à celle obtenue avec la composition de l'exemple 1.

EXEMPLE 6

On prépare les compositions suivantes :

COMPOSITION (A) :

| | | | |
|---|---|---|---|
| - Dihydroxyacétone | | | 5,0 g |
| - Alcool éthylique | | | 15,0 g |
| - Gomme de xanthane | | | 1,0 g |
| - Conservateur | qs | | |
| - Triéthanolamine | qs | pH = 8,5 | |
| - Eau déminéralisée | | qsp | 100,0 g |

COMPOSITION (B) :

| | | | |
|---|---|---|---|
| - 5,6-dihydroxyindole | | | 2,0 g |
| - Alcool éthylique | | | 15,0 g |
| - Gomme de xanthane | | | 1,0 g |
| - Conservateur | qs | | |
| - Triéthanolamine | qs | pH = 8,5 | |
| - Eau déminéralisée | | qsp | 100,0 g |

La coloration de la peau peut être obtenue selon les différents modes d'applications suivants :

- on applique la composition (A) puis la même quantité en poids de composition (B) sur la peau;
- on applique la composition (B) puis la même quantité en poids de composition (A) sur la peau.

La coloration obtenue selon l'un quelconque de ces deux modes d'application est équivalente à celle obtenue avec la composition de l'exemple 1.

EXEMPLE 7

On prépare la composition suivante :

| | | | |
|---|---|---|---|
| - Dihydroxyacétone | | | 2,0 g |
| - 5,6-dihydroxyindole | | | 0,4 g |
| - Alcool éthylique | | | 10,0 g |
| - Gomme de xanthane | | | 1,0 g |
| - Conservateur | qs | | |
| - NaOH | qs | pH = 7 | |
| - Eau déminéralisée | | qsp | 100,0 g |

Cette composition permet d'obtenir sur peau non bronzée, une coloration identique à celle d'un bronzage naturel.

EXEMPLE 8

On prépare les compositions suivantes :

COMPOSITION (A) :

Dans une première étape, on prépare un constituant (A1) comprenant les vésicules. On fond, en agitant doucement à une température de 95°C, un mélange de 3,8 g de lipides non-ioniques, de formule :

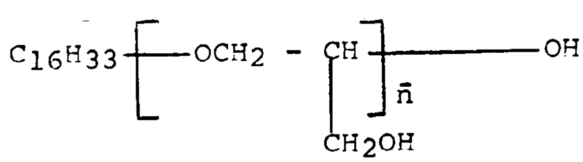

où $\bar{n}$ est une valeur statistique moyenne égale à 3, avec 3,8 g de cholestérol, 0,4 g de sel monosodique du glutamate de stéaroyle vendu sous la dénomination ACYLGLUTAMATE HS 11 par la Société AJINOMOTO.

On introduit dans le mélange fondu 16 g d'eau portée à 90°C, contenant un conservateur et l'on mélange pendant environ 5 minutes. A la phase ainsi obtenue, on ajoute 24 g d'eau à 20°C; on agite le mélange quelques minutes puis on complète par 2 g d'eau à 20°C et on affine le mélange par un passage dans un homogénéiseur

haute pression à 500 bars (Rannie).

Dans une seconde étape, on prépare un constituant (A2) comprenant la phase aqueuse de dispersion :

| | | |
|---|---|---|
| - Dihydroxyacétone | | 3,5 g |
| - Paraméthoxycinnamate de 2-éthylhexyle vendu sous la dénomination ESCALOL 557 par la Société VAN DIK | | 2,0 g |
| - 2-hydroxy 4-méthoxybenzophénone | | 0,5 g |
| - Glycérol | | 2,0 g |
| - Huile de vaseline | | 8,0 g |
| - Huile de silicone | | 5,0 g |
| - Hydroxyéthylcellulose vendue sous la dénomination NATROSOL PLUS GRADE 330 CS par la Société AQUALON | | 0,5 g |
| - Conservateurs, parfum | qs | |
| - Eau déminéralisée | qsp | 50,0 g |

On réalise le mélange du constituant (A1) et du constituant (A2) et on homogénéise sous agitation douce.

- pH spontané du mélange (A1)+(A2) = 5,1

COMPOSITION (B) :

On prépare la composition (B) de la même façon que la composition (A) :

Lipides non-ioniques de formule : 3,8 g

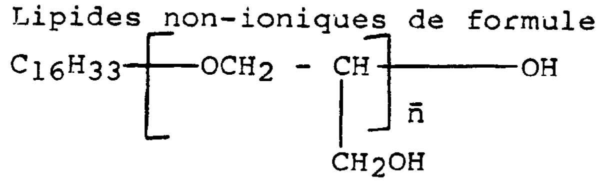

où $\bar{n}$ est une valeur statistique moyenne égale à 3,

- sel monosodique du glutamate de stéaroyle, vendu sous la dénomination ACYLGLUTAMATE HS 11 par la Société AJINOMOTO 0,4 g
- Cholestérol 3,8 g
- Glycérol 3,0 g
- Huile de vaseline 15,0 g
- Hydroxyéthylcellulose vendue sous la dénomination NATROSOL PLUS GRADE 330 CS par la Société AQUALON 0,5 g
- 3-méthyl 5,6-dihydroxyindole 0,2 g
- Conservateur qs
- pH spontané 6,4
- Eau déminéralisée qsp 100,0 g

La coloration de la peau peut être obtenue selon les différents modes d'application suivants :

- on applique la composition (A) puis la même quantité en poids de la composition (B) sur la peau;
- on applique la composition (B) puis la même quantité en poids de la composition (A) sur la peau.

La coloration obtenue sur une peau non bronzée selon l'un quelconque de ces deux modes d'application est identique à celle d'un bronzage naturel.

EXEMPLE 9

On prépare les compositions suivantes :

COMPOSITION (A) :

Le mode de préparation est identique à celui de l'exemple 8.

| | | |
|---|---|---|
| Lipides non-ioniques de formule : | | 3,8 g |

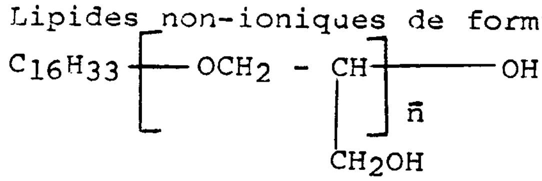

| | | |
|---|---|---|
| où $\bar{n}$ est une valeur statistique moyenne égale à 3, | | |
| - sel monosodique du glutamate de stéaroyle, vendu sous la dénomination ACYLGLUTAMATE HS 11 par la Société AJINOMOTO | | 0,4 g |
| - Cholestérol | | 3,8 g |
| - Dihydroxyacétone | | 5,0 g |
| - Paraméthoxycinnamate de 2-éthylhexyle vendu sous la dénomination ESCALOL 557 par la Société VAN DIK | | 2,0 g |
| - 2-hydroxy 4-méthoxybenzophénone | | 0,5 g |
| - Glycérol | | 2,0 g |
| - Huile de vaseline | | 8,0 g |
| - Huile de silicone | | 5,0 g |
| - Hydroxyéthylcellulose vendue sous la dénomination NATROSOL PLUS GRADE 330 CS par la Société AQUALON | | 0,5 g |
| - Conservateurs, parfum qs | | |
| - pH spontané 6,2 | | |
| - Eau déminéralisée | qsp | 100,0 g |

COMPOSITION (B) :

On prépare un émulsion solaire huile-dans-eau ayant la composition suivante :

| | | | |
|---|---|---|---|
| - 5,6-dihydroxyindole | | 1,2 | g |
| - Mélange d'alcool cétylstéarylique et d'alcool cétylstéarylique oxyéthyléné à 33 moles d'oxyde d'éthylène, vendu par la Société HENKEL sous la dénomination SINNOWAX AO | | 7,0 | g |
| - Mélange de monostéarate et de distéarate de glycéryle non auto-émulsionnable | | 2,0 | g |
| - Alcool cétylique | | 1,5 | g |
| - Huile de silicone | | 1,5 | g |
| - Huile de vaseline | | 15,0 | g |
| - Glycérine | | 20,0 | g |
| - Parfum, conservateur qs | | | |
| - pH spontané 5,8 | | | |
| - Eau | qsp | 100,0 | g |

La coloration de la peau peut être obtenue selon les différents modes d'application suivants :

- on applique la composition (A) puis la même quantité en poids de la composition (B) sur la peau;
- on applique la composition (B) puis la même quantité en poids de la composition (A) sur la peau.

La coloration obtenue sur une peau non bronzée selon l'un quelconque de ces deux modes d'application est identique à celle d'un bronzage naturel.

EXEMPLE 10

On prépare les compositions suivantes :

COMPOSITION (A) :

Le mode de préparation est identique à celui de l'exemple 8.

Lipides non-ioniques de formule : 3,8 g

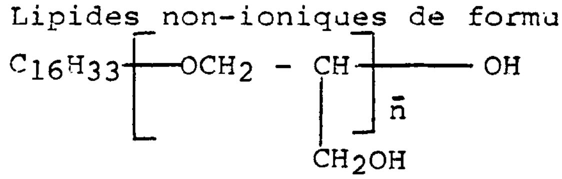

où n̄ est une valeur statistique moyenne égale à 3,

- sel monosodique du glutamate de stéaroyle, vendu sous la dénomination ACYLGLUTAMATE HS 11 par la Société AJINOMOTO 0,4 g
- Cholestérol 3,8 g
- Dihydroxyacétone 5,0 g
- Paraméthoxycinnamate de 2-éthylhexyle vendu sous la dénomination ESCALOL 557 par la Société VAN DIK 2,0 g
- 2-hydroxy 4-méthoxybenzophénone 0,5 g
- Glycérol 2,0 g
- Huile de vaseline 8,0 g
- Huile de silicone 5,0 g
- Hydroxyéthylcellulose vendue sous la dénomination NATROSOL PLUS GRADE 330 CS par la Société AQUALON 0,5 g
- Conservateurs, parfum qs
- pH spontané 6,2
- Eau déminéralisée qsp 100,0 g

COMPOSITION (B) :

- 2-méthyl 5,6-dihydroxyindole,HBr 0,2 g
- Alcool éthylique qsp 100,0 g

La coloration de la peau peut être obtenue selon les différents modes d'application suivants :

- on applique la composition (A) puis la même quantité en poids de la composition (B) sur la peau;
- on applique la composition (B) puis la même quantité en poids de la composition (A) sur la peau.

La coloration obtenue sur 'une peau non bronzée selon l'un quelconque de ces deux modes d'application est identique à celle d'un bronzage naturel.

EXEMPLE 11

On prépare les compositions suivantes :

COMPOSITION (A) :

Le mode de préparation est identique à celui de l'exemple 8.

| | | |
|---|---|---|
| Lipides non-ioniques de formule : | | 3,8 g |

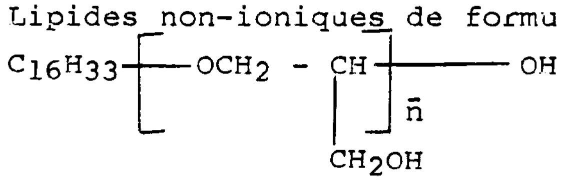

où $\bar{n}$ est une valeur statistique moyenne égale à 3,

| | | |
|---|---|---|
| - sel monosodique du glutamate de stéaroyle, vendu sous la dénomination ACYLGLUTAMATE HS 11 par la Société AJINOMOTO | | 0,4 g |
| - Cholestérol | | 3,8 g |
| - Dihydroxyacétone | | 5,0 g |
| - Paraméthoxycinnamate de 2-éthylhexyle vendu sous la dénomination ESCALOL 557 par la Société VAN DIK | | 2,0 g |
| - 2-hydroxy 4-méthoxybenzophénone | | 0,5 g |
| - Glycérol | | 2,0 g |
| - Huile de vaseline | | 8,0 g |
| - Huile de silicone | | 5,0 g |
| - Hydroxyéthylcellulose vendue sous la dénomination NATROSOL PLUS GRADE 330 CS par la Société AQUALON | | 0,5 g |
| - Conservateurs, parfum qs | | |
| - pH spontané 6,2 | | |
| - Eau déminéralisée | qsp | 100,0 g |

COMPOSITION (B) :

| | | |
|---|---|---|
| - 2,3-diméthyl 5,6-dihydroxyindole | | 0,3 g |
| - Alcool éthylique | qsp | 100,0 g |

La coloration de la peau peut être obtenue selon les différents modes d'application suivants :

- on applique la composition (A) puis la même quantité en poids de la composition (B) sur la peau;
- on applique la composition (B) puis la même quantité en poids de la composition (A) sur la peau.

La coloration obtenue sur une peau non bronzée selon l'un quelconque de ces deux modes d'application est identique à celle d'un bronzage naturel.

**Revendications**

1. Composition destinée à conférer à la peau une coloration similaire à la coloration résultant d'un bronzage naturel, caractérisée par le fait qu'elle est constituée par l'association de :
   a) un composant (A) contenant dans un milieu approprié pour une application topique, de la dihydroxy acétone; et
   b) un composant (B) contenant dans un milieu approprié pour une application topique, un dérivé indolique répondant à la formule :

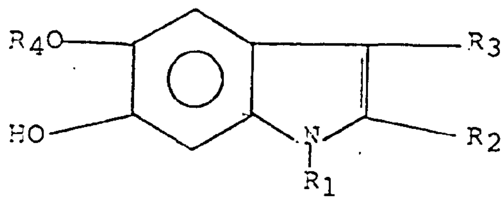

(I)

   dans laquelle $R_1$, $R_2$, $R_3$, $R_4$ désignent, indépendamment l'un de l'autre, hydrogène ou un groupement alkyle en $C_1$-$C_4$, à la condition que lorsque $R_4$ représente un groupement alkyle en $C_1$-$C_4$, $R_1$, $R_2$, $R_3$ désignent hydrogène, ainsi que les sels d'addition de ces composés;
   les composants (A) et (B) faisant l'objet d'une application simultanée, successive ou décalée dans le temps.

2. Composition selon la revendication 1, caractérisée par le fait que le dérivé indolique de formule (I) est choisi parmi le 5,6-dihydroxyindole, le 2-méthyl 5,6-dihydroxyindole, le 3-méthyl 5,6-dihydroxy indole, le 2,3-diméthyl 5,6-dihydroxyindole.

3. Composition selon la revendication 1 ou 2, caractérisée par le fait que les composants (A) et (B) constituent une seule composition contenant 0,1 à 10% en poids et de préférence 1 à 6% en poids de dihydroxyacétone et 0,1 à 10% en poids, de préférence 0,1 à 5% en poids de dérivé indolique de formule (I), ces pourcentages étant exprimés par rapport au poids total de la composition.

4. Composition selon la revendication 1 ou 2, caractérisée par le fait que les composants (A) et (B) sont séparés, le composant (A) contenant la dihydroxy acétone dans des proportions comprises entre 0,1 et 10% en poids et en particulier entre 1 et 6% en poids par rapport au poids total du compcsant (A), le composant (B) contenant le dérivé indolique de formule (I) dans des proportions comprises entre 0,1 et 10% en poids et de préférence entre 0,1 et 5% en poids par rapport au poids total du composant (B).

5. Composition selon l'une quelconque des revendications 1 à 4, caractérisée par le fait que le rapport en poids entre le dérivé indolique de formule (I) et la dihydroxyacétone est supérieur à 0,01.

6. Composition selon l'une quelconque des revendications 1 à 5, caractérisée par le fait que le milieu approprié pour l'application topique est un milieu comprenant de l'eau, un mélange d'eau et d'un ou plusieurs solvant(s) organique(s), un mélange de solvants organiques ou des corps gras, ces derniers et les solvants organiques étant cosmétiquement acceptables.

7. Composition selon l'une quelconque des revendications 1 à 6, caractérisée par le fait que les solvants sont choisis parmi les alcools inférieurs en $C_1$-$C_4$, les monoalcools saturés à longue chaîne ayant 10 à 18 atomes de carbone, les polyols ayant 2 à 8 atomes de carbone, les alkylèneglycols, les éthers de glycols, les esters acétiques de monoalkyl($C_1$-$C_3$)éthers de l'éthylèneglycol, les esters d'acides gras saturés ayant 14 à 16 atomes de carbone et d'alcools saturés ayant 1 à 4 atomes de carbone.

8. Composition selon l'une quelconque des revendications 1 à 7, caractérisée par le fait que les solvants sont choisis parmi l'alcool éthylique, l'alcool cétylique, le propylèneglycol, l'éthylèneglycol mono éthyléther et l'éthylèneglycol monobutyléther.

9. Composition selon l'une quelconque des revendications 1 à 8, caractérisée par le fait que les corps gras sont choisis parmi les huiles minérales, végétales ou animales, les acides gras, les alcools gras, les esters d'acides gras, les triglycérides d'acides gras.

10. Composition selon l'une quelconque des revendications 1 à 9, caractérisée par le fait que les milieux appropriés pour une application topique comportent des épaississants.

11. Composition selon l'une quelconque des revendications 1 à 10, caractérisée par le fait que le pH du composant (A) est compris entre 3 et 10 et de préférence entre 3 et 7 et que le pH du composant (B) est compris entre 3 et 10 et de préférence entre 4 et 7.

12. Composition selon l'une quelconque des revendications 1 à 11, caractérisée par le fait que les composants (A) et (B) se présentent sous forme de crèmes, de liquides plus ou moins épaissis, de dispersions vésiculaires de lipides amphiphiles ioniques ou non ioniques, de compositions aérosols ou d'huiles.

13. Composition selon l'une quelconque des revendications 1 à 12, caractérisée par le fait que l'un au moins des constituants (A) et (B) contient au moins un filtre solaire filtrant le rayonnement UV-A et/ou UV-B.

14. Composition selon l'une quelconque des revendications 1 à 13, caractérisée par le fait que les composants (A) et (B) contiennent en plus des agents tensio-actifs, des agents hydratants, adoucissants, opacifiants, des vitamines, des parfums ou des agents conservateurs.

15. Dispositif à plusieurs compartiments ou "kit" ou "nécessaire" pouvant conférer à la peau une coloration similaire à la coloration résultant d'un bronzage naturel, caractérisé par le fait qu'il comprend un premier compartiment contenant le composant (A) renfermant, dans un milieu approprié pour une application topique, la dihydroxyacétone et un second compartiment contenant le composant (B) renfermant dans un milieu approprié pour une application topique, au moins un dérivé indolique de formule (I) défini dans la revendication 1.

16. Dispositif selon la revendication 15, caractérisé par le fait qu'il est constitué par un dispositif à deux poches séparées réunies dans un étui souple, la première poche renfermant le composant (A) tel que céfini dans la revendication 1, et la seconde poche renfermant le composant (B) tel que défini dans la revendication 1.

17. Procédé cosmétique de coloration de la peau afin de lui conférer une coloration similaire à celle d'un bronzage naturel, caractérisé par le fait que l'on applique sur la peau la composition telle que définie dans l'une quelconque des revendications 1 à 14.

## Claims

1. Composition intended for imparting to the skin a coloration similar to the coloration resulting from natural tanning, characterized in that it consists of the combination of:
   a) a component (A) containing dihydroxyacetone in a medium suitable for topical application; and
   b) a component (B) containing, in a medium suitable for topical application, an indole derivative corresponding to the formula:

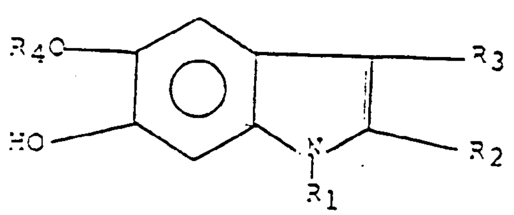

(I)

in which $R_1$, $R_2$, $R_3$ and $R_4$ denote, independently of one another, hydrogen or a $C_1$-$C_4$-alkyl group, on condition that, when $R_4$ represents a $C_1$-$C_4$-alkyl group, $R_1$, $R_2$ and $R_3$ denote hydrogen; as well as the addition salts of these compounds;

the components (A) and (B) being applied simultaneously, successively or separated by an interval of time.

2. Composition according to Claim 1, characterized in that the indole derivative of the formula (I) is selected from 5,6-dihydroxyindole, 2-methyl-5,6-dihydroxyindole, 3-methyl-5,6-dihydroxyindole and 2,3-dimethyl-5,6-dihydroxyindole.

3. Composition according to Claim 1 or 2, characterized in that the components (A) and (B) constitute a single composition containing 0.1 to 10 % by weight, and preferably 1 to 6 % by weight, of dihydroxyacetone, and 0.1 to 10 % by weight, and preferably 0.1 to 5 % by weight, of indole derivative of the formula (I), these percentages being expressed relative to the total weight of the composition.

4. Composition according to Claim 1 or 2, characterized in that the components (A) and (B) are separated, the component (A) containing dihydroxyacetone in proportions of between 0.1 and 10 % by weight, and especially between 1 and 6 % by weight, relative to the total weight of the component (A), and the component (B) containing the indole derivative of the formula (I) in proportions of between 0.1 and 10 % by weight, and preferably between 0.1 and 5 % by weight, relative to the total weight of the component (B).

5. Composition according to any one of Claims 1 to 4, characterized in that the ratio by weight of the indole derivative of the formula (I) to dihydroxyacetone is greater than 0.01.

6. Composition according to any one of Claims 1 to 5, characterized in that the medium suitable for topical application is a medium comprising water, a mixture of water and one or more organic solvent(s), a mixture of organic solvents or fats, the latter and the organic solvents being cosmetically acceptable.

7. Composition according to any one of Claims 1 to 6, characterized in that the solvents are selected from $C_1$-$C_4$ lower alcohols, saturated long-chain monohydric alcohols having 10 to 18 carbon atoms, polyols having 2 to 8 carbon atoms, alkylene glycols, glycol ethers, acetic acid esters of ethylene glycol ($C_1$-$C_3$)-monoalkyl ethers and esters of saturated fatty acids having 14 to 16 carbon atoms and saturated alcohols having 1 to 4 carbon atoms.

8. Composition according to any one of Claims 1 to 7, characterized in that the solvents are selected from ethyl alcohol, cetyl alcohol, propylene glycol, ethylene glycol monoethyl ether and ethylene glycol monobutyl ether.

9. Composition according to any one of Claims 1 to 8, characterized in that the fats are selected from mineral, vegetable or animal oils, fatty acids, fatty alcohols, fatty acid esters and fatty acid triglycerides.

10. Composition according to any one of Claims 1 to 9, characterized in that the media suitable for topical application contain thickeners.

11. Composition according to any one of Claims 1 to 10, characterized in that the pH of the component (A) is between 3 and 10 and preferably between 3 and 7, and in that the pH of the component (B) is between 3 and 10 and preferably between 4 and 7.

12. Composition according to any one of Claims 1 to 11, characterized in that the components (A) and (B) are present in the form of creams, more or less thickened liquids, vesicular dispersions of ionic or nonionic amphiphilic lipids, aerosol compositions or oils.

13. Composition according to any one of Claims 1 to 12, characterized in that at least one of the constituents (A) and (B) contains at least one sunscreen agent screening out UV-A and/or UV-B radiation.

14. Composition according to any one of Claims 1 to 13, characterized in that the components (A) and (B) contain, in addition, surfactants, hydrating agents, emollients, opacifiers, vitamins, fragrances or preservatives.

15. Multi-compartment device or "kit" or "outfit" capable of imparting to the skin a coloration similar to the coloration resulting from natural tanning, characterized in that it comprises a first compartment containing the component (A) containing dihydroxyacetone in a medium suitable for topical application, and a second compartment containing the component (B) containing at least one indole derivative of the formula (I), defined in Claim 1, in a medium suitable for topical application.

16. Device according to Claim 15, characterized in that it consists of a device comprising two separate bags combined in a flexible case, the first bag containing the component (A) as defined in Claim 1, and the second bag containing the component (B) as defined in Claim 1.

17. Cosmetic process for coloring the skin in order to impart thereto a coloration similar to that of a natural tan, characterized in that the composition as defined in any one of Claims 1 to 14 is applied to the skin.

## Patentansprüche

1. Zusammensetzung, die dazu bestimmt ist, der Haut eine Färbung zu verleihen, die der Färbung entspricht, die bei einer natürlichen Bräunung entsteht, dadurch gekennzeichnet, daß sie durch die Kombination
   a) einer Komponente (A), die in einem zur lokalen Anwendung geeigneten Medium Dihydroxyaceton enthält, und
   b) einer Komponente (B), die in einem zur lokalen Anwendung geeigneten Medium ein Indol-Derivat der Formel (I):

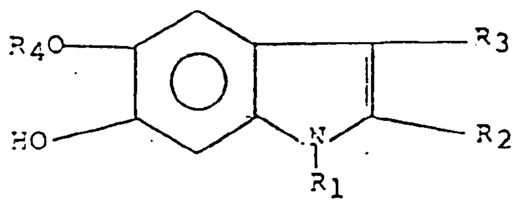

(I)

   in der $R_1$, $R_2$, $R_3$ und $R_4$ unabhängig voneinander Wasserstoff oder eine $C_{1-4}$-Alkylgruppe bezeichnen, unter der Bedingung, daß, wenn $R_4$ eine $C_{1-4}$-Alkylgruppe darstellt, $R_1$, $R_2$, $R_3$ Wasserstoff bezeichnen, enthält; sowie Additionssalzen dieser Verbindungen gebildet wird,
   wobei die Komponenten (A) und (B) gleichzeitig, nacheinander oder zeitversetzt angewendet werden.

2. Zusammensetzung nach Anspruch 1, dadurch gekennzeichnet, daß das Indolderivat der Formel (I) 5,6-Dihydroxyindol, 2-Methyl-5,6-dihydroxyindol, 3-Methyl-5,6-dihydroxyindol, 2,3-Dimethyl-5,6-dihydroxyindol ausgewählt ist.

3. Zusammensetzung nach Anspruch 1 oder 2, dadurch gekennzeichnet daß die Komponenten (A) und (B) eine einzige Zusammensetzung bilden, die 0,1 bis 10 Gew.-%, und vorzugsweise 1 bis 6 Gew.-% Dihydroxyaceton und 0,1 bis 10 Gew.-%, vorzugsweise 0,1 bis 5 Gew.-% Indolderivat der Formel (I) enthält, wobei sich die Prozentangaben auf das Gesamtgewicht der Zusammensetzung beziehen.

4. Zusammensetzung nach Anspruch 1 oder 2, dadurch gekennzeichnet daß die Komponenten (A) und (B) getrennt sind; die Komponente (A), die Dihydroxyaceton umfaßt, dieses in Mengen zwischen 0,1 und 10 Gew.-% und insbesondere zwischen 1 und 6 Gew.-%, bezogen auf das Gesamtgewicht der Komponente (A) enthält; die Komponente (B), die das Indolderivat der Formel (I) umfaßt, dieses in Mengen zwischen 0,1 und 10 Gew.-% und vorzugsweise zwischen 0,1 und 5 Gew.-%, bezogen auf das Gesamtgewicht der Komponente (B), enthält.

5. Zusammensetzung nach einem der Ansprüche 1 bis 4, dadurch gekennzeichnet daß das Gewichtsverhältnis zwischen dem Indolderivat der Formel (I) und dem Dihydroxyaceton über 0,01 liegt.

6. Zusammensetzung nach einem der Ansprüche 1 bis 5, dadurch gekennzeichnet daß das zur lokalen Anwendung geeignete Medium ein Medium ist, das Wasser, ein Gemisch aus Wasser und einem oder mehreren organischen Lösungsmittel(n), ein Gemisch aus organischen Lösungsmitteln oder Fettstoffen enthält, wobei diese letztgenannten sowie die organischen Lösungsmittel kosmetisch akzeptabel sind.

7. Zusammensetzung nach einem der Ansprüche 1 bis 6, dadurch gekennzeichnet daß die Lösungsmittel aus den niederen $C_{1-4}$-Alkoholen; den langkettigen, gesättigten Monoalkoholen mit 10 bis 18 Kohlenstoffatomen; den Polyolen mit 2 bis 8 Kohlenstoffatomen; den Alkylenglykolen; den Etherglykolen; den Essigsäureestern von Monoalkyl($C_1$-$C_3$)ethern von Ethylenglykol; den Estern aus gesättigten Fettsäuren mit 14 bis 16 Kohlenstoffatomen und gesättigten Alkoholen mit 1 bis 4 Kohlenstoffatomen ausgewählt sind.

8. Zusammensetzung nach einem der Ansprüche 1 bis 7, dadurch gekennzeichnet daß die Lösungsmittel aus Ethylalkohol, Cetylalkohol, Propylenglykol, Ethylenglykolmonoethylether und Ethylenglykolmonobutylether ausgewählt sind.

9. Zusammensetzung nach einem der Ansprüche 1 bis 8, dadurch gekennzeichnet, daß die Fettstoffe aus den mineralischen, pflanzlichen oder tierischen Ölen, den Fettsäuren, den Fettalkoholen, den Fettsäureestern, den Triglyceriden von Fettsäuren ausgewählt sind.

10. Zusammensetzung nach einem der Ansprüche 1 bis 9, dadurch gekennzeichnet, daß die für eine lokale Anwendung geeigneten Medien Verdickungsmittel enthalten.

11. Zusammensetzung nach einem der Ansprüche 1 bis 10, dadurch gekennzeichnet, daß der pH der Komponente (A) zwischen 3 und 10 und vorzugsweise zwischen 3 und 7 liegt und daß der pH der Komponente (B) zwischen 3 und 10 und vorzugsweise zwischen 4 und 7 liegt.

12. Zusammensetzung nach einem der Ansprüche 1 bis 11, dadurch gekennzeichnet, daß die Komponenten (A) und (B) in Form von Cremes, von mehr oder weniger dicken Flüssigkeiten, von bläschenförmigen Dispersionen amphiphiler ionischer oder nicht-ionischer Fette, von Aerosol-Zusammensetzungen oder von Ölen vorhanden sind.

13. Zusammensetzung nach einem der Ansprüche 1 bis 12, dadurch gekennzeichnet, daß mindestens eine der Komponenten einen Sonnenfilter enthält, der die UV-A und/oder UV-B-Strahlung filtert.

14. Zusammensetzung nach einem der Ansprüche 1 bis 13, dadurch gekennzeichnet, daß die Komponenten (A) und (B) darüber hinaus oberflächenaktive Mittel, Feuchtigkeitspendende Mittel, glättende Substanzen, deckende Substanzen, Vitamine, Parfums oder Konservierungsmittel enthalten.

15. Set mit mehreren Fächern, oder "kit" oder "Necessaire", das der Haut eine Färbung verleihen kann, die gleich der Färbung ist, die bei einer natürlichen Bräunung entsteht, dadurch gekennzeichnet, daß es ein erstes Fach aufweist, welches die Komponente (A), die Dihydroxyaceton enthält, in einem zur lokalen Anwendung geeigneten Medium eingeschlossen enthält, und es ein zweites Fach aufweist, welches die Komponente (B) in einem zur lokalen Anwendung geeigneten Medium eingeschlossen enthält, wobei die Komponente (B) mindestens ein Indolderivat der Formel (I), das in Anspruch 1 definiert ist, umfaßt.

16. Set nach Anspruch 15, dadurch gekennzeichnet, daß es aus einer Anordnung von zwei getrennten Taschen, die sich zusammen in einem biegsamen Etui befinden, besteht, wobei die erste Tasche die Komponente (A), wie sie in Anspruch 1 definiert ist, beinhaltet und die zweite Tasche die Komponente (B), wie sie in Anspruch 1 definiert ist, beinhaltet.

17. Kosmetisches Verfahren zum Färben der Haut, um ihr eine Färbung zu verleihen, die der bei einer natürlichen Bräunung entspricht, dadurch gekennzeichnet, daß man die Zusammensetzung, wie sie in einem der Ansprüche 1 bis 14 definiert ist, auf die Haut aufträgt.